# EUROPEAN PATENT APPLICATION

(11) **EP 3 754 031 A1**
(43) Date of publication of application: **23.12.2020**
(21) Application number: 19754232.7
(22) Date of filing: 07.02.2019
(51) Int. Cl.: C12Q 1/6886, C12Q 1/6841, G01N 33/53, G01N 33/574

(54) **BIOMARKER FOR PREDICTING EFFECTS OF ANTI-PD-1 ANTIBODY/ANTI-PD-L1 ANTIBODY THERAPY**

(30) Priority: 14.02.2018 JP 2018024556
(71) Applicant: National University Corporation Tokai National Higher Education and Research System, Nagoya-shi Aichi 464-8601 (JP)
(72) Inventor: MIYAI Yuki, Nagoya-shi, Aichi 464-8601 (JP); ENOMOTO Atsushi, Nagoya-shi, Aichi 464-8601 (JP); TAKAHASHI Masahide, Nagoya-shi, Aichi 464-8601 (JP); HASEGAWA Yoshinori, Nagoya-shi, Aichi 464-8601 (JP); HASE Tetsunari, Nagoya-shi, Aichi 464-8601 (JP); ANDO Yuichi, Nagoya-shi, Aichi 464-8601 (JP)
(74) Representative: Steinecke, Peter
(86) International application number: PCT/JP2019/004521
(87) International publication number: WO 2019/159825

(57) **Abstract**

An object of the present invention is to provide a novel marker that can be used to predict the effect of anti-PD-1 antibody/anti-PD-L1 antibody therapy with high sensitivity, and use thereof. Provided is a biomarker for predicting the effect of anti-PD-1 antibody/anti-PD-L1 antibody therapy, including an immunoglobulin superfamily containing leucine-rich repeat (ISLR).

## Description

### [Technical Field]

The present invention relates to a biomarker useful for predicting the effect of anti-PD-1 (programmed cell death protein-1) antibody/anti-PD-L1 (programmed death-ligand 1) antibody therapy, and use thereof. The present application claims priority based on Japanese Patent Application No. 2018-024556 filed on February 14, 2018, the entire contents of which are incorporated herein by reference.

### [Background Art]

Anti-PD-1 antibody therapy is used for the treatment of many malignancies such as malignant melanoma, non-small cell lung cancer, renal cell carcinoma, head and neck cancer, gastric cancer, classic Hodgkin lymphoma, urothelial carcinoma, and malignant pleural mesothelioma (for example, see NPLs 1 and 2). Anti-PD-Ll antibody therapy is also used for the treatment of Merkel cell carcinoma and non-small cell lung cancer. Many clinical trials are in progress, and the range of indications is considered to expand increasingly in the future. However, there are problems such as high economic toxicity and the presence of patients who do not respond to anti-PD-1 antibody drugs/anti-PD-L1 antibody drugs (non-responders), and there is an urgent need to develop markers for predicting the effects of anti-PD-1 antibody drugs/anti-PD-L1 antibody drugs.

Currently, the companion diagnostic drug PD-L1 (programmed death-ligand 1) is used as an effect prediction marker on a commercial basis when pembrolizumab is used for the treatment of non-small cell lung cancer, but has been proved to actually have a mechanism of action different from the originally assumed one (NPL 3). The possibility that PD-L1 may be inappropriate as a clinically used biomarker is increasing. In fact, there are cases for which anti-PD-1 antibody/anti-PD-L1 antibody therapy is recognized to be effective regardless of the presence or absence of PD-L1 expression (NPLs 4, 5, and 9), and there are also false negative/false positive problems.

Tumor-infiltrating lymphocytes (TILs), Immunoscore (registered trademark) (see NPL 8), total tumor mutation burden (TMB), mutation-associated neoantigens (MANAs), serum markers, and the like, which are currently-examined markers, involve a problem such as complicated procedures, high costs, or limited application targets. Therefore, they have not been put into practical use, except the case where pembrolizumab was approved for the treatment of solid cancer having high microsatellite instability (MSI-High) in December, 2018.

### [Citation List]

### [Patent Literature]

[PTL 1] WO 2017/022472
[PTL 2] US 2005/0260639 A1

### [Non Patent Literature]

[NPL 1] Package insert of OPDIVO (registered trademark) I.V. Infusion 20 mg/OPDIVO (registered trademark) I.V. Infusion 100 mg, revised 21st edition (November, 2018)
[NPL 2] Package insert of KEYTRUDA (registered trademark) Injection 20 mg/KEYTRUDA (registered trademark) Injection 100 mg, revised 9th edition (December, 2018)
[NPL 3] Hui E, Cheung J, Zhu J, Su X, Taylor MJ, Wallweber HA, Sasmal DK, Huang J, Kim JM, Mellman I, et al. (2017) T cell costimulatory receptor CD28 is a primary target for PD-1-mediated inhibition. Science 355(6332), 1428-1433.
[NPL 4] Brahmer J, Reckamp KL, Baas P, Crino L, Eberhardt WE, Poddubskaya E, Antonia S, Pluzanski A, Vokes EE, Holgado E, et al. (2015) Nivolumab versus docetaxel in advanced squamous-cell non small-cell lung cancer. New England Journal of Medicine 373, 123-135.
[NPL 5] Kang YK, Boku N, Satoh T, Ryu MH, Chao Y, Kato K, Chung HC, Chen JS, Muro K, Kang WK, et al. (2017) Nivolumab in patients with advanced gastric or gastro-oesophageal junction cancer refractory to, or intolerant of, at least two previous chemotherapy regimens (ONO-4538-12, ATTRACTION-2): a randomised, double-blind, placebo-controlled, phase 3 trial. Lancet 390(10111), 2461-2471.
[NPL 6] Walter K, Omura N, Hong SM, Griffith M, Vincent A, Borges M, Goggins M. (2010) Overexpression of smoothened activates the sonic hedgehog signaling pathway in pancreatic cancer-associated fibroblasts. Clinical Cancer Research. 16(6), 1781-9.
[NPL 7] Allinen M, Beroukhim R, Cai L, Brennan C, Lahti-Domenici J, Huang H, Porter D, Hu M, Chin L, Richardson A, et al. (2004) Molecular characterization of the tumor microenvironment in breast cancer. Cancer cell. 6(1), 17-32.
[NPL 8] Galon J, Costes A, Sanchez-Cabo F, Kirilovsky A, Mlecnik B, Lagorce-Pages C, Tosolini M, Camus M, Berger A, Wind P, et al. (2006). Type, density, and location of immune cells within human colorectal tumors predict clinical outcome. Science. 313(5795), 1960-4.
[NPL 9] Fehrenbacher L, Spira A, Ballinger M, Kowanetz M, Vansteenkiste J, Mazieres J, Park K, Smith D, Artal-Cortes A, Lewanski C, Braiteh F, Waterkamp D, He P, Zou W, Chen DS, Yi J, Sandler A, Rittmeyer A; POPLAR Study Group. (2016). Atezolizumab versus docetaxel for patients with previously treated non-small-cell lung cancer (POPLAR): a multicentre, open-label, phase 2 randomised controlled trial. Lancet. 387(10030), 1837-46

### [Summary of Invention]

### [Technical Problem]

As described above, the advent of a novel biomarker that can be used to predict the effect of anti-PD-1 antibody/anti-PD-L1 antibody therapy has been earnestly desired. Therefore, an object of the present invention is to provide a novel marker that can be used to predict the effect of anti-PD-1 antibody/anti-PD-L1 antibody therapy with high sensitivity, and use thereof.

### [Solution to Problem]

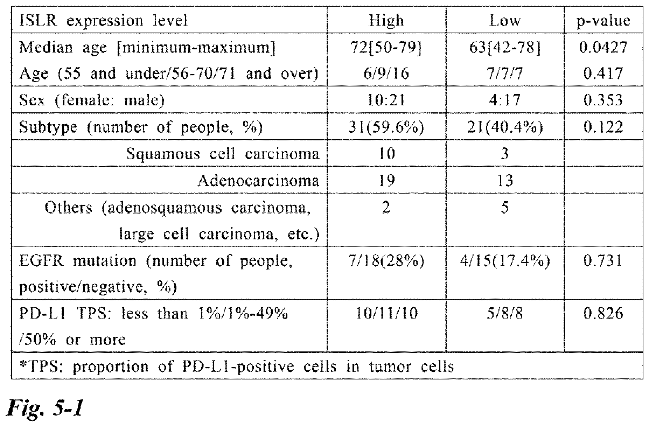

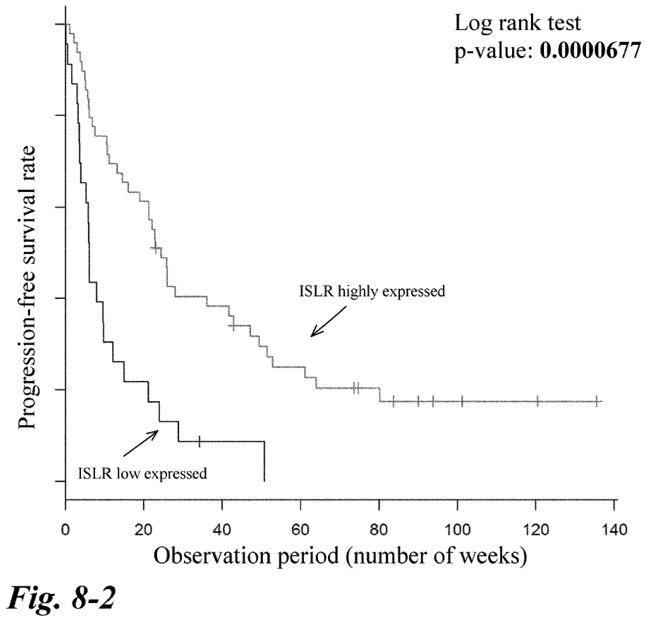

While advancing research to solve the above problems, the present inventors focused on an immunoglobulin superfamily containing leucine-rich repeat (ISLR). It is known that ISLR is expressed in cancer-associated fibroblasts of lung cancer, breast cancer, pancreatic cancer, and the like (one of stromal cells of tumor tissues) (PTLs 1 and 2 and NPLs 6 and 7), but the significance, function, and the like of the expression of ISLR in cancer-associated fibroblasts are unknown. Therefore, the present inventors conducted various experiments using clinical specimens of lung cancer, focusing on the relationship between the expression of ISLR in cancer-associated fibroblasts and the effect of anti-PD-1 antibody/anti-PD-L1 antibody therapy. As a result of detailed studies (see the Examples below), a high correlation was observed between the expression of ISLR and the effect of anti-PD-1 antibody/anti-PD-L1 antibody therapy. That is, it was found that the expression of ISLR in cancer-associated fibroblasts is extremely useful as a marker for predicting the effect of anti-PD-1 antibody/anti-PD-L1 antibody therapy. Regarding the relationship between the expression of ISLR and the prediction of therapeutic effect, the anti-PD-1 antibody therapy and the anti-PD-Ll antibody therapy showed similar tendencies (Figs. 5-1 to 8-2).

In view of the facts that the anti-PD-1 antibody/anti-PD-L1 antibody therapy is widely applied to malignant melanoma, non-small cell lung cancer, renal cell cancer, head and neck cancer, gastric cancer, Hodgkin lymphoma, urothelial cancer, malignant pleural mesothelioma, and the like, that there is a certain commonality in the development/progression of these cancers/malignant tumors, and further that the expression of ISLR is observed in cancer-associated fibroblasts of lung cancer, breast cancer, pancreatic cancer, and colon cancer, ISLR can be reasonably expected to function as an effect prediction marker similarly in various cancers/malignant tumors to which the anti-PD-1 antibody/anti-PD-L1 antibody therapy can be applied.

The following inventions are based on the above results and observations.
[1] A biomarker for predicting the effect of anti-PD-1 antibody/anti-PD-L1 antibody therapy, including an immunoglobulin superfamily containing leucine-rich repeat (ISLR).
[2] A method for predicting the effect of anti-PD-1 antibody/anti-PD-L1 antibody therapy, involving using expression of ISLR in cells in a tumor tissue derived from a subject as an index.
[3] The method according to [2], wherein the cells are nucleated cells other than tumor cells.
[4] The method according to [2], wherein the cells are fibroblasts.
[5] The effect prediction method according to any one of [2] to [4], including the following steps (1) to (3):
   (1) a step of preparing a tumor tissue specimen collected from a subject;
   (2) a step of examining expression of ISLR in cells in the tumor tissue specimen; and
   (3) a step of predicting the effect of anti-PD-1 antibody/anti-PD-L1 antibody therapy based on the result of step (2), wherein high expression of ISLR indicates that the anti-PD-1 antibody/anti-PD-L1 antibody therapy can be expected to be effective.
[6] The effect prediction method according to [5], wherein, in step (2), the expression level of ISLR is determined based on the number of ISLR-positive cells and/or the ratio of ISLR-positive cells to ISLR-negative cells.
[7] The effect prediction method according to [5] or [6], further including the following step (4):
   (4) a step of excluding a subject for whom the anti-PD-1 antibody/anti-PD-L1 antibody therapy cannot be expected to be effective from treatment targets based on the prediction result.
[8] The effect prediction method according to [5] or [6], further including the following step (4'):
   (4') a step of determining or changing a treatment policy for a subject based on the prediction result.
[9] The effect prediction method according to any one of [2] to [8], wherein the subject is a patient suffering from malignant melanoma, non-small cell lung cancer, renal cell carcinoma, head and neck cancer, gastric cancer, Hodgkin lymphoma, urothelial cancer, breast cancer, pancreatic cancer, or colon cancer.
[10] A kit for predicting the effect of anti-PD-1 antibody/anti-PD-L1 antibody therapy, including an ISLR detection reagent.
[11] The effect prediction kit according to [10], wherein the ISLR detection reagent is an *in situ* hybridization probe.
[12] The effect prediction kit according to [10], wherein the ISLR detection reagent is an anti-ISLR antibody.

### [Brief Description of Drawings]

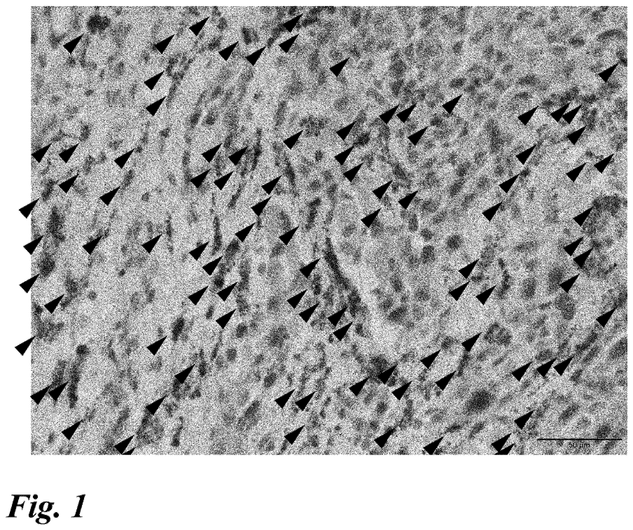

[Fig. 1] Expression of ISLR in the tumor stroma using a surgical specimen of lung cancer. The expression of ISLR was examined by RNA *in situ* hybridization. The cells indicated by black arrowheads are ISLR-expressing cells. This specimen has high expression of ISLR.

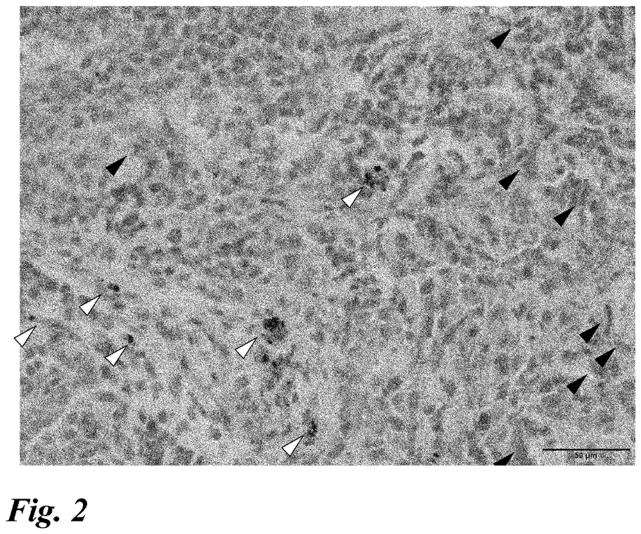

[Fig. 2] Expression of ISLR in the tumor stroma using a surgical specimen of lung cancer. The expression of ISLR was examined by RNA *in situ* hybridization. The cells indicated by black arrowheads are ISLR-expressing cells. The cells indicated by white arrowheads are charcoal phagocytes that are also observed in normal tissues. This specimen has low expression of ISLR.

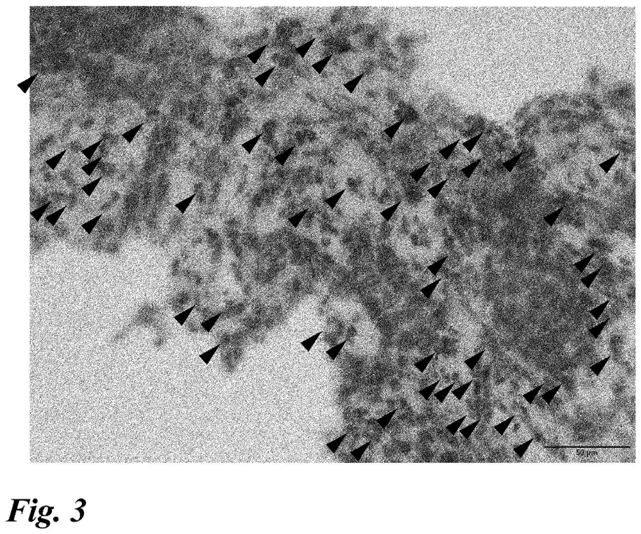

[Fig. 3] Expression of ISLR in the tumor stroma using a biopsy specimen in the diagnosis of lung cancer. The expression of ISLR was examined by RNA *in situ* hybridization. The cells indicated by black arrowheads are ISLR-expressing cells. This specimen has high expression of ISLR.

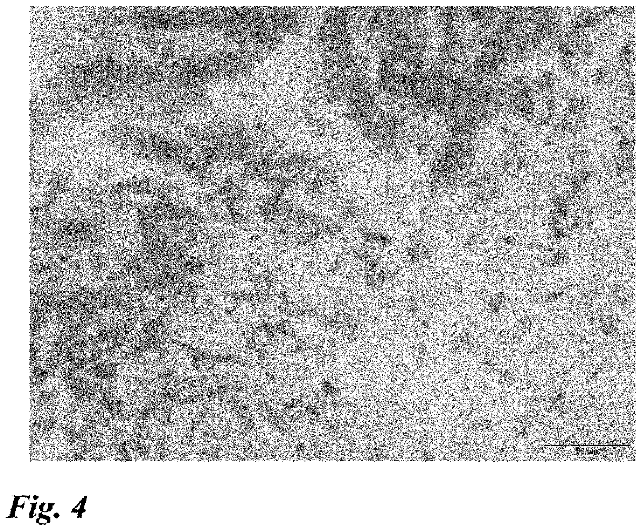

[Fig. 4] Expression of ISLR in the tumor stroma using a biopsy specimen in the diagnosis of lung cancer. The expression of ISLR was examined by RNA *in situ* hybridization. No ISLR-expressing cell could be confirmed in this specimen.
[Fig. 5-1] A table in which patients (only patients who received anti-PD-1 antibody therapy) are divided into two groups according to the level (high or low) of ISLR expression in the tumor stroma. No bias was observed in age bracket, sex, subtype, EGFR mutation-positive lung cancer, or PD-L1 expression level between the two groups with high and low ISLR expression levels. However, the group with high expression of ISLR includes elderly people more than those included in the group with low expression of ISLR. Note that TPS is a proportion of PD-L1-positive cells in tumor cells.

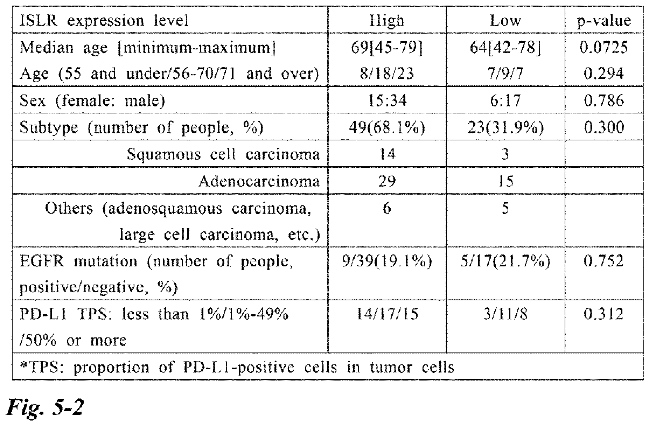

[Fig. 5-2] A table in which patients (including patients who received the anti-PD-1 antibody therapy and patients who received the anti-PD-Ll antibody therapy) are divided into two groups according to the level (high or low) of ISLR expression in the tumor stroma. No bias was observed in any of age, sex, subtype, EGFR mutation-positive cancer, and PD-L1 expression level in a population also including patients who received the anti-PD-Ll antibody therapy. Note that TPS is a proportion of PD-L1-positive cells in tumor cells.

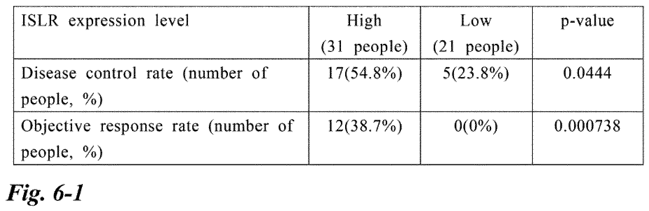

[Fig. 6-1] Correlation between the level (high or low) of ISLR expression in the tumor stroma and the effect (evaluation in patients who received the anti-PD-1 antibody therapy). It is shown that the group with high expression of ISLR has significantly high disease control rate and objective response rate as compared with the group with low expression of ISLR.

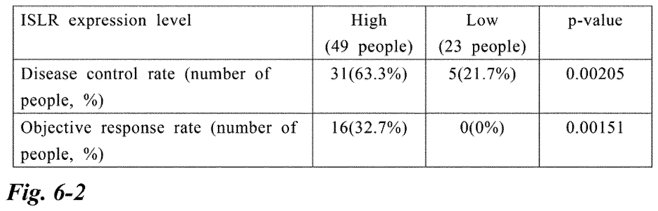

[Fig. 6-2] Correlation between the level (high or low) of ISLR expression in the tumor stroma and the effect (evaluation in patients including patients who received the anti-PD-1 antibody therapy and patients who received the anti-PD-Ll antibody therapy). It is shown that the group with high expression of ISLR has significantly high disease control rate and objective response rate as compared with the group with low expression of ISLR. Note that 7 out of 9 patients who received the anti-PD-Ll antibody therapy had high expression of ISLR, that a therapeutic effect was observed in 3 patients, and that disease control was attained in 7 patients. In contrast, 2 patients with low expression of ISLR (all patients) could not obtain a therapeutic effect.

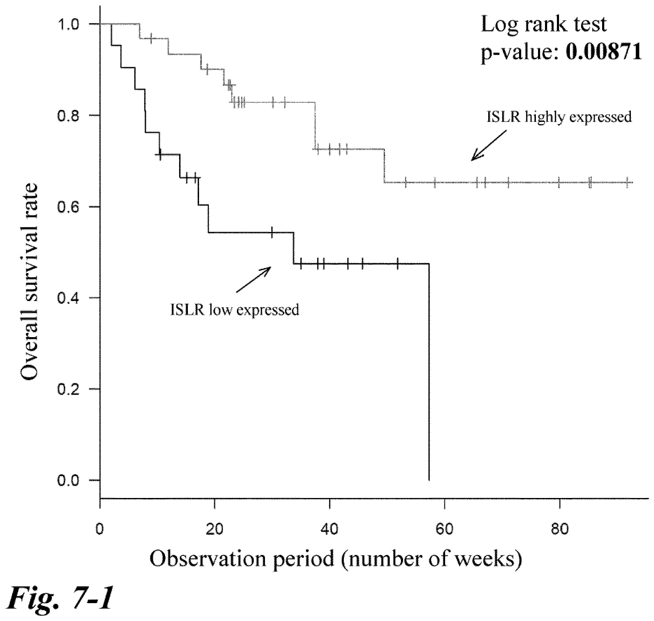

[Fig. 7-1] A result (graph) of evaluating an overall survival period based on the level (high or low) of ISLR expression (evaluation in patients who received the anti-PD-1 antibody therapy). It is shown that the group with high expression of ISLR shows a significantly extended overall survival period as compared with the group with low expression of ISLR. Note that the overall survival period is a period from the start of follow-up (that is, the day on which the anti-PD-1 antibody therapy was started) to death (regardless of the reason therefor).

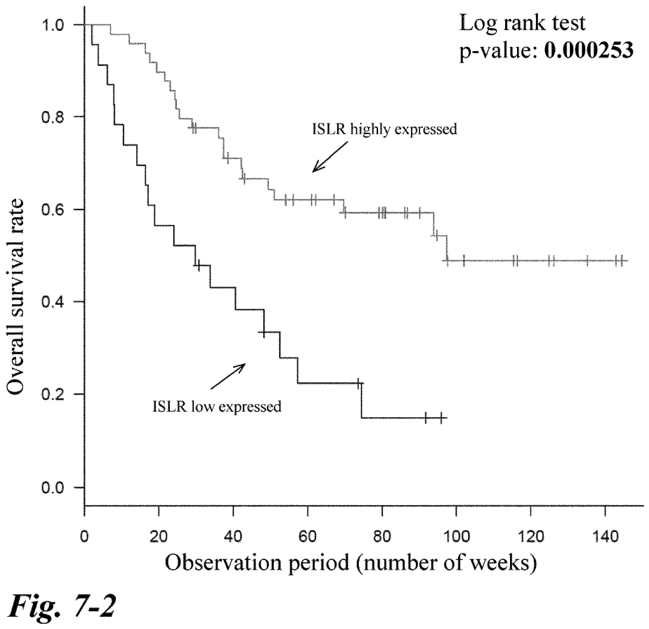

[Fig. 7-2] A result (graph) of evaluating the overall survival period based on the level (high or low) of ISLR expression (evaluation in patients including patients who received the anti-PD-1 antibody therapy and patients who received the anti-PD-Ll antibody therapy). It is shown that the group with high expression of ISLR shows a significantly extended overall survival period as compared with the group with low expression of ISLR. Note that the overall survival period is a period from the start of follow-up (that is, the day on which anti-PD-1 antibody/anti-PD-L1 antibody therapy was started) to death (regardless of the reason therefor).

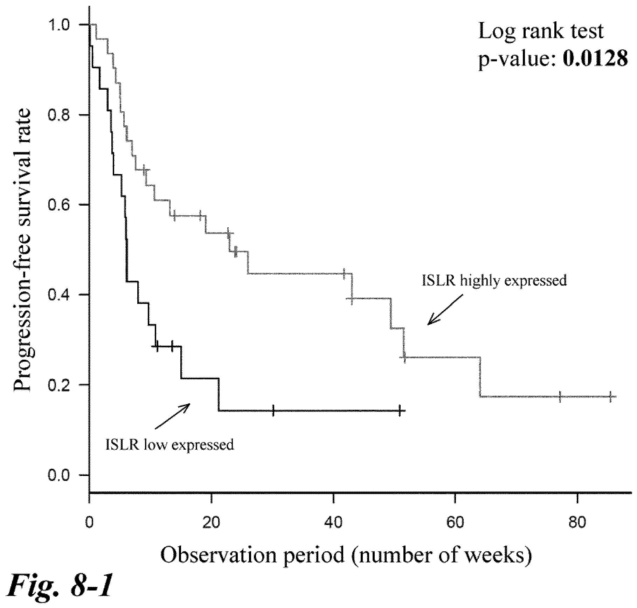

[Fig. 8-1] A result (graph) of evaluating a progression-free survival period based on the level (high or low) of ISLR expression (evaluation in patients who received the anti-PD-1 antibody therapy). It is shown that the group with high expression of ISLR shows a significantly extended progression-free survival period as compared with the group with low expression of ISLR. Note that the progression-free survival period is a period from the start of follow-up (that is, the day on which the anti-PD-1 antibody therapy was started) to the exacerbation of the disease state (appearance of a new lesion, increase in size of a lesion to be evaluated, or death).
[Fig. 8-2] A result (graph) of evaluating the progression-free survival period based on the level (high or low) of ISLR expression (evaluation in patients including patients who received the anti-PD-1 antibody therapy and patients who received the anti-PD-Ll antibody therapy). It is shown that the group with high expression of ISLR shows a significantly extended progression-free survival period as compared with the group with low expression of ISLR. Note that the progression-free survival period is a period from the start of follow-up (that is, the day on which an anti-PD-1 antibody/anti-PD-L1 antibody therapy was started) to the exacerbation of the disease state (appearance of a new lesion, increase in size of a lesion to be evaluated, or death).

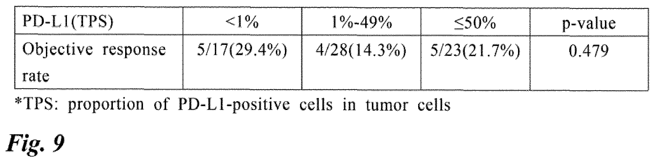

[Fig. 9] A table showing the relationship between the expression level of PD-L1 in cancer cells and the effect. The effect was observed at all the expression levels (i.e., there is no correlation between the expression level and the effect). Note that TPS is a proportion of PD-L1-positive cells in tumor cells.

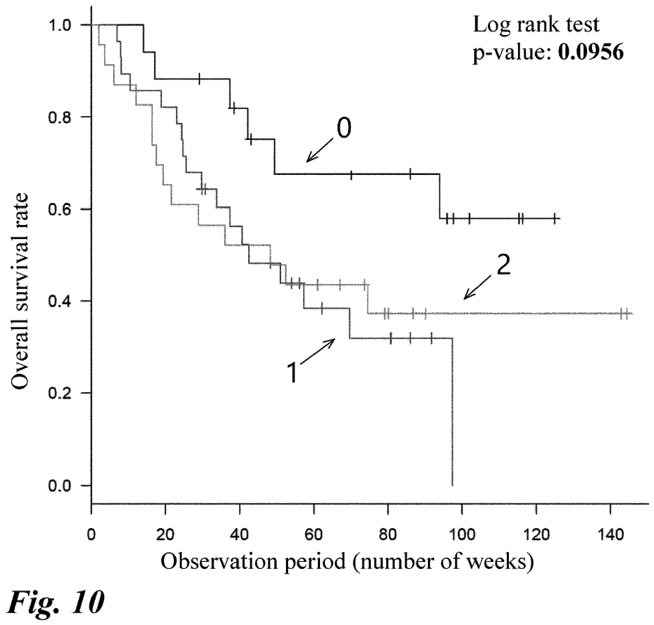

[Fig. 10] A result (graph) of evaluating the overall survival period based on the expression level of PD-L1. There was no association between the expression level of PD-L1 and the overall survival period.

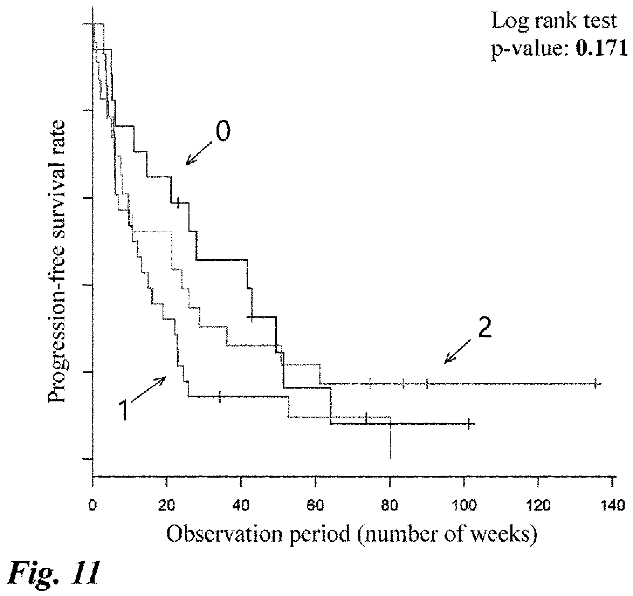

[Fig. 11] A result (graph) of evaluating the progression-free survival period based on the expression level of PD-L1. There was no association between the expression of PD-L1 and the progression-free survival period.

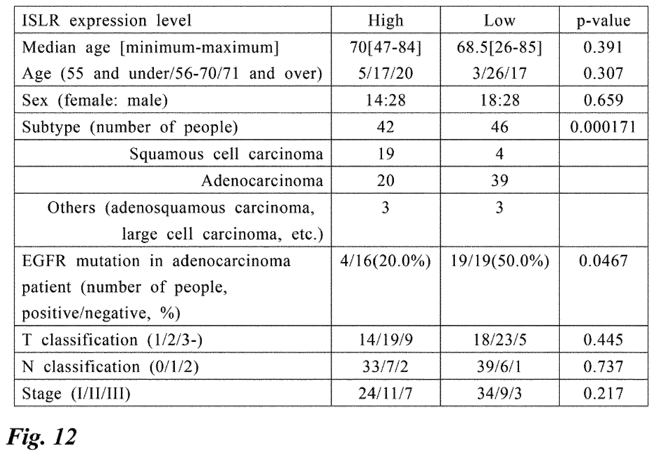

[FIG. 12] A table in which patients (not including patients who received the anti-PD-1 antibody therapy or the anti-PD-Ll antibody therapy) are divided into two groups according to the level (high or low) of ISLR expression in the tumor stroma. Between the two groups with high and low levels of ISLR expression, no bias was observed in age, sex, T classification or N classification of the TNM classification, or stage. However, the group with high expression of ISLR has less adenocarcinoma and less EGFR mutant-positive lung cancer than those of the group with low expression of ISLR.

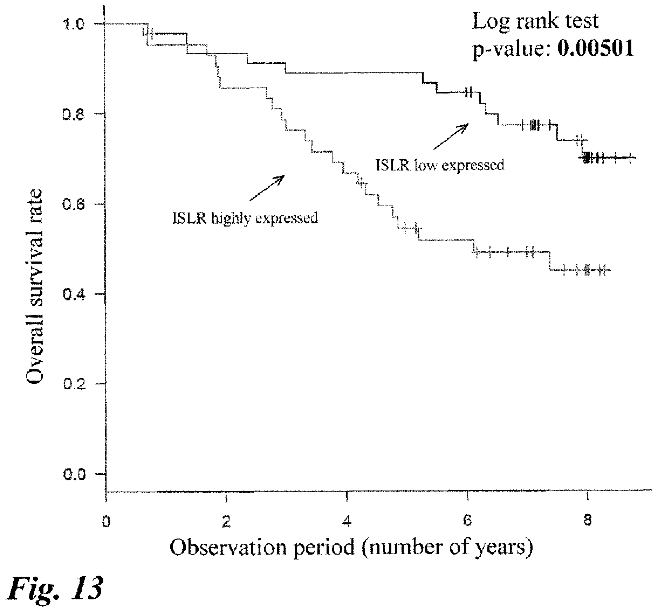

[FIG. 13] A result (graph) of evaluating the overall survival period based on the level (high or low) of ISLR expression. It is shown that the group with high expression of ISLR shows a significantly short overall survival period as compared with the group with low expression of ISLR. Note that the overall survival period is a period from the start of follow-up (in this case, the day on which surgery was performed) to death (regardless of the reason therefor).

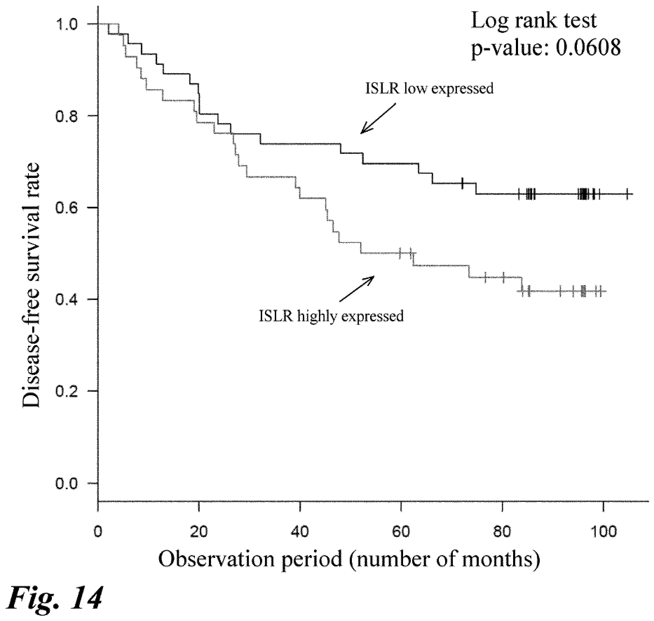

[FIG. 14] A result (graph) of evaluating a disease-free survival period based on the level (high or low) of ISLR expression. There was no significant association between the expression of ISLR and the disease-free survival period. Note that the disease-free survival period is a period from the start of follow-up (in this case, the day on which surgery was performed) to recurrence/death (regardless of the reason therefor).

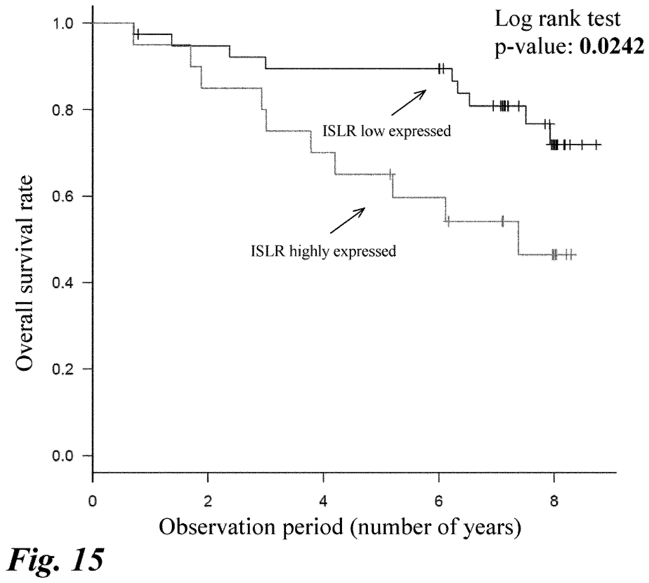

[FIG. 15] A result (graph) of evaluating the overall survival period only for adenocarcinoma based on the level (high or low) of ISLR expression. It is shown that the group with high expression of ISLR shows a significantly short overall survival period as compared with the group with low expression of ISLR. Note that the overall survival period is a period from the start of follow-up (in this case, the day on which surgery was performed) to death (regardless of the reason therefor).

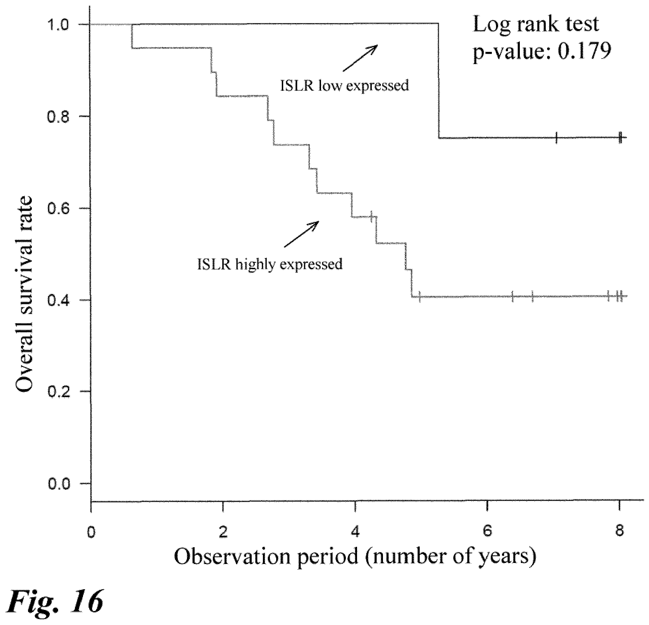

[FIG. 16] A result (graph) of evaluating the overall survival period only for squamous cell carcinoma based on the level (high or low) of ISLR expression. There was no significant association between the expression of ISLR and the overall survival period. Note that the overall survival period is a period from the start of follow-up (in this case, the day on which surgery was performed) to death (regardless of the reason therefor).

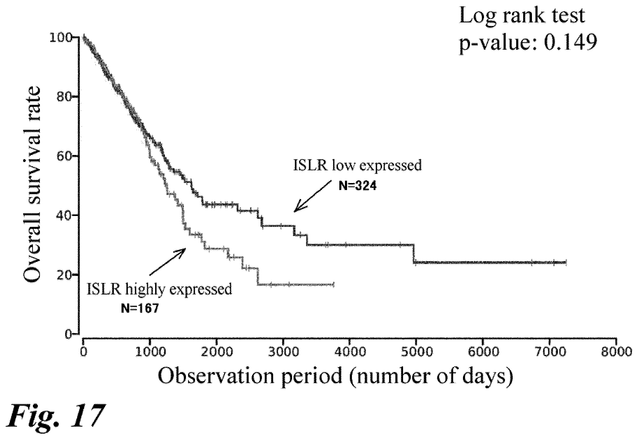

[FIG. 17] A result (graph) of evaluating the overall survival period of pulmonary adenocarcinoma patients registered in TCGA based on the level (high or low) of ISLR expression. There was no significant association between the expression of ISLR and the overall survival period. Note that the overall survival period is a period from the start of follow-up to death (regardless of the reason therefor).

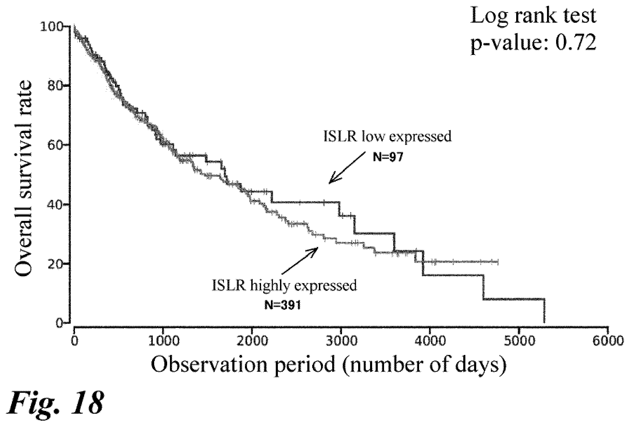

[FIG. 18] A result (graph) of evaluating the overall survival period of squamous cell lung cancer patients registered in TCGA based on the level (high or low) of ISLR expression. There was no significant association between the expression of ISLR and the overall survival period. Note that the overall survival period is a period from the start of follow-up to death (regardless of the reason therefor).

### [Description of Embodiments]

### 1. Biomarker for predicting effect of anti-PD-1 antibody/anti-PD-L1 antibody therapy

A first aspect of the present invention relates to a marker molecule whose expression has been found to correlate with the effect of anti-PD-1 antibody/anti-PD-L1 antibody therapy, i.e., a "biomarker for predicting the effect of anti-PD-1 antibody/anti-PD-L1 antibody therapy". The "biomarker for predicting the effect of anti-PD-1 antibody/anti-PD-L1 antibody therapy" refers to a biomolecule useful for predicting the effect of a treatment method using an anti-PD-1 antibody drug or anti-PD-L1 antibody drug (referred to as "anti-PD-1 antibody/anti-PD-L1 antibody therapy" herein). The "biomarker for predicting the effect of anti-PD-1 antibody/anti-PD-L1 antibody therapy" of the present invention (hereinafter, sometimes abbreviated as "marker of the present invention") can be used to predict the effect of anti-PD-1 antibody/anti-PD-L1 antibody therapy, that is, to determine whether the anti-PD-1 antibody/anti-PD-L1 antibody therapy can be expected to be therapeutically effective (whether the anti-PD-1 antibody/anti-PD-L1 antibody therapy exhibits a therapeutic effect). Therefore, the marker of the present invention provides an objective decision material for distinguishing between patients who respond to the anti-PD-1 antibody/anti-PD-L1 antibody therapy and patients who do not respond thereto. In particular, the marker of the present invention is highly useful in identifying or selecting patients who do not respond to the anti-PD-1 antibody/anti-PD-L1 antibody therapy (non-responders).

As used herein, the "biomolecule" refers to a molecule (compound) found in a living body. A specific biomolecule is used as a biomarker in the present invention, and, in the use thereof (typically, application thereof to an effect prediction method), the biomolecule in a specimen/sample separated from a living body is used.

The marker of the present invention includes an immunoglobulin superfamily containing leucine-rich repeat (ISLR). ISLR is a cell membrane-bound or secretory molecule, and is recognized to be highly expressed in lung cancer, breast cancer, pancreatic cancer, and the like (see, for example, PTL 2 and NPLs 6 and 7).

The amino acid sequence of ISLR and the gene sequence encoding it, which are registered in a public database, are shown in the attached sequence listing. The correspondence between the sequence numbers and the sequences is as follows.

SEQ ID NO: 1: amino acid sequence of ISLR (NCBI Reference Sequence: NP_005536.1, immunoglobulin superfamily containing leucine-rich repeat protein precursor [Homo sapiens].)

SEQ ID NO: 2: cDNA sequence of ISLR (GeneID:3671, NCBI Reference Sequence: NM_005545.3, Homo sapiens immunoglobulin superfamily containing leucine-rich repeat (ISLR), transcript variant 1, mRNA.)

For ISLR, the sequences of a variant (variant 2) (Homo sapiens immunoglobulin superfamily containing leucine-rich repeat (ISLR), transcript variant 2, mRNA, amino acid sequence: NP_958934.1, and cDNA sequence: NM_201526.1) are known. The amino acid sequence of the variant is the same as the above amino acid sequence (SEQ ID NO: 1).

The anti-PD-1 antibody/anti-PD-L1 antibody therapy is used for the treatment of various cancers/malignant tumors, as described above. For example, anti-PD-1 antibody drugs such as nivolumab (trade name "OPDIVO (registered trademark)") and pembrolizumab (trade name "KEYTRUDA (registered trademark)"), and anti-PD-Ll antibody drugs such avelumab (trade name "BAVENCIO (registered trademark)"), atezolizumab (trade name "TECENTRIQ (registered trademark)") and durvalumab (trade name "IMFINZI (registered trademark)") are clinically applied.

The term "effect of anti-PD-1 antibody/anti-PD-L1 antibody therapy" includes the effect of a treatment method using an anti-PD-1 antibody drug/anti-PD-L1 antibody drug alone, as well as the effect of a combination therapy using an anti-PD-1 antibody drug/anti-PD-L1 antibody drug and a medicament other than the anti-PD-1 antibody drug/anti-PD-L1 antibody drug. Therefore, the present invention can also be used to predict the effect of a combination therapy using an anti-PD-1 antibody drug/anti-PD-L1 antibody drug and a drug other than the anti-PD-1 antibody drug/anti-PD-L1 antibody drug (for example, use of nivolumab and ipilimumab (anti-CTLA-4 antibody drug) in combination). In other words, the present invention is also intended for use as a means for identifying or selecting patients who are not compatible with the combination therapy (for whom the combination therapy cannot be expected to be therapeutically effective).

### 2. Method for predicting effect of anti-PD-1 antibody/anti-PD-L1 antibody therapy

A second aspect of the present invention relates to use of the marker of the present invention, and provides a method for predicting the effect of anti-PD-1 antibody/anti-PD-L1 antibody therapy (hereinafter, sometimes referred to as "the effect prediction method of the present invention"). The effect prediction method of the present invention involves predicting the effect of anti-PD-1 antibody/anti-PD-L1 antibody therapy using expression of ISLR in cells in a tumor tissue derived from a subject as an index. Typically, the following steps (1) to (3) are carried out:
(1) a step of preparing a tumor tissue specimen collected from a subject;
(2) a step of examining expression of ISLR in cells in the tumor tissue specimen; and
(3) a step of predicting the effect of anti-PD-1 antibody/anti-PD-L1 antibody therapy based on the result of step (2), wherein high expression of ISLR indicates that the anti-PD-1 antibody/anti-PD-L1 antibody therapy can be expected to be effective.

### Step (1)

In step (1), a tumor tissue specimen derived from a subject is prepared. The subject is a patient to whom the anti-PD-1 antibody/anti-PD-L1 antibody therapy is scheduled or examined to be applied. Therefore, usually, the subject is a patient suffering from a disease to which the anti-PD-1 antibody/anti-PD-L1 antibody therapy can be applied, for example, malignant melanoma, non-small cell lung cancer, renal cell carcinoma, head and neck cancer, gastric cancer, Hodgkin lymphoma, urothelial cancer, breast cancer, pancreatic cancer, or colon cancer. A tumor tissue (containing cancer cells and fibroblasts) collected from the subject is used as a specimen. For example, a tumor tissue collected for pathological examination/diagnosis (generally called biopsy sample, biopsy tissue, or the like) or a tumor tissue collected during surgery (generally called surgical specimen, surgical material, or the like) can be used as a specimen. The tumor tissue is collected from a primary site (primary lesion) or a metastatic lesion. The specimen is prepared prior to carrying out the present invention. That is, the effect prediction method of the present invention does not include treatment or operation on a patient (collection/isolation of a tumor tissue) to prepare a specimen.

A tumor tissue in which tumor cells and fibroblasts coexist is used as a specimen. If nucleated cells other than tumor cells are present in the tumor tissue, it is presumed that fibroblasts also exist, and the tumor tissue can be used as a specimen.

### Step (2)

In step (2), expression of ISLR in cells in the tumor tissue specimen is examined. Since the tumor tissue specimen contains tumor cells, it is preferable to identify nucleated cells other than the tumor cells in the tumor tissue specimen and to examine the expression of ISLR in the cells. In addition, cells remaining after excluding tumor cells, lymphocytes, granulocytes, vascular endothelial cells, and macrophages from all nucleated cells may be regarded as a population of fibroblasts (all fibroblasts), and the expression of ISLR in the population may be examined. In order to examine the "expression of ISLR", detection targeting ISLR mRNA or ISLR protein is performed. That is, the expression of ISLR mRNA or ISLR protein is detected. In the present invention, qualitative or quantitative detection of the expression of ISLR mRNA or ISLR protein is performed.

The expression of ISLR mRNA can be detected by various methods using a primer or probe specific to ISLR mRNA, such as *in situ* hybridization, RT-PCR, quantitative PCR, and Northern blotting. On the other hand, when the expression of ISLR protein is detected, it is advisable to use a substance showing specific bindingness to ISLR protein and to adopt an immunological technique (for example, immunohistochemistry). The immunological technique enables highly sensitive detection. Also, the operation is relatively convenient. Antibodies are usually used as the substances showing specific bindingness to ISLR protein, but any substances having specific bindingness to ISLR and whose binding can be detected or measured can be used without being limited to antibodies.

In immunohistochemical staining of living tissues, generally, the operations of (1) fixation/paraffin-embedding (or frozen-embedding), (2) deparaffinization (not necessary in the case of frozen-embedding), (3) a primary antibody reaction, (4) addition of a labeling reagent, (5) a color development reaction, and (6) dehydration/clearing/embedding are performed in order. If necessary, antigen activation treatment, endogenous peroxidase removal treatment (when peroxidase is used as a labeling substance), inhibition of a nonspecific reaction (treatment with a bovine serum albumin solution or the like), or the like is performed prior to the primary antibody reaction. In addition, if necessary, nuclear staining (for example, Mayer's hematoxylin can be used) is performed before dehydration/clearing/embedding. Regarding immunohistochemical staining methods of living tissues, various literatures and books can be referred to (for example, "Enzyme Antibody Method, revised 3rd edition", edited by Keiichi Watanabe and Kazuho Nakane, Gakusai Kikaku K.K.).

### Step (3)

In step (3), the effect of anti-PD-1 antibody/anti-PD-L1 antibody therapy is predicted based on the result of step (2). The phrase "predicting the effect of anti-PD-1 antibody/anti-PD-L1 antibody therapy" has the same meaning as determining whether a therapeutic effect is obtained or the possibility that a therapeutic effect may be obtained, when the anti-PD-1 antibody/anti-PD-L1 antibody therapy is performed. In the present invention, the index or determination criterion that "high expression of ISLR indicates that the anti-PD-1 antibody/anti-PD-L1 antibody therapy can be expected to be effective" is adopted, based on the fact that the expression of ISLR in cells in tumor tissue specimens and the therapeutic effect obtained by the anti-PD-1 antibody/anti-PD-L1 antibody showed a positive correlation, in the study using clinical specimens. Therefore, typically, the anti-PD-1 antibody/anti-PD-L1 antibody therapy is determined to exhibit an effect based on high expression of ISLR in cells in tumor tissue specimens, and determined to exhibit no effect based on low expression of ISLR in cells in tumor tissue specimens. However, as in the following example, the probability (possibility) that the anti-PD-1 antibody/anti-PD-L1 antibody therapy may exhibit an effect may be determined according to the expression level (degree).

The expression level of ISLR can be determined, for example, using the number of ISLR-positive cells or the ratio of ISLR-positive cells to ISLR-negative cells alone, or using them in combination. Hereinafter, an example of a method for determining the expression level will be illustrated.

Tumor tissues are observed (e.g., macroscopically observed with an optical microscope), and those in which the proportion of ISLR-positive cells in all nucleated cells from which tumor cells, lymphocytes, granulocytes, vascular endothelial cells, and macrophages are excluded is a certain value (for example, a numerical value within the range of 10% to 25%, specifically, 10%, 15%, 20%, or 25%) or more are defined as having a "high expression level", and those in which the proportion thereof is less than the value are defined as having a "low expression level". It is determined that "the anti-PD-1 antibody/anti-PD-L1 antibody therapy exhibits an effect" when the expression level is high, and that "the anti-PD-1 antibody/anti-PD-L1 antibody therapy does not exhibit an effect" when the expression level is low.

The proportions of ISLR-positive cells may be classified into three or more ranks (for example, three, four, or five ranks) to make a quantitative determination. A specific example of this aspect (an example in the case where the proportions are classified into four ranks) is as follows. The expression level is determined as 1 when the proportion of ISLR-positive cells is less than 5%; determined as 2 when the proportion of ISLR-positive cells is 5% or more and less than 10%; determined as 3 when the proportion of ISLR-positive cells is 10% or more and less than 15%; and determined as 4 when the proportion of ISLR-positive cells is 15% or more and less than 20%. The probability that the anti-PD-1 antibody/anti-PD-L1 antibody therapy may exhibit an effect is determined as less than 20% when the expression level is 1; determined as 20% or more and less than 50% when the expression level is 2; determined as 50% or more and less than 80% when the expression level is 3; and determined as 80% or more when the expression level is 4.

The reference value for determining the level (high or low) of ISLR expression, the expression level classes, the determination result associated with each of the classes, and the like can be set through preliminary experiments and the like. Note that the determination/evaluation in the present invention can be automatically/mechanically made without depending on the decision made by a person having specialized knowledge such as a doctor or a laboratory technician.

The prediction result according to the present invention provides useful information in identifying/selecting persons who do not respond to the anti-PD-1 antibody/anti-PD-L1 antibody therapy. Therefore, in one aspect of the present invention, a subject for whom the anti-PD-1 antibody/anti-PD-L1 antibody therapy cannot be expected to be effective is excluded from treatment targets based on the prediction result (step (4)). The exclusion of a subject for whom the anti-PD-1 antibody/anti-PD-L1 antibody therapy cannot be expected to be effective from treatment targets has the same meaning as identifying or selecting a subject for whom the anti-PD-1 antibody/anti-PD-L1 antibody therapy can be expected to be effective as a treatment target. Therefore, a patient with whom the anti-PD-1 antibody/anti-PD-L1 antibody therapy is compatible is identified or selected by this step.

The prediction result can also be used for determining or changing a treatment policy for the patient. Therefore, in another aspect of the present invention, a treatment policy for the subject is determined or changed based on the prediction result (step (4')). The treatment policy is designed or selected according to the prediction result. Typically, the application of the anti-PD-1 antibody/anti-PD-L1 antibody therapy can be recommended to the subject (patient) if it is predicted that the anti-PD-1 antibody/anti-PD-L1 antibody therapy can be expected to be therapeutically effective. On the other hand, when it is predicted that the anti-PD-1 antibody/anti-PD-L1 antibody therapy cannot be expected to be therapeutically effective, the subject (patient) should be excluded from the targets for treatment with the anti-PD-1 antibody/anti-PD-L1 antibody therapy, and any other therapy (e.g., conventional drug therapy, radiation therapy, palliative care, surgery, or immunotherapy) is recommended.

As described above, the present invention can be used to form a more appropriate treatment policy for each patient. As a result, unnecessary treatment is not required, so that benefits such as reduction of the burden on patients, avoidance of side effects, and reduction of medical costs can be obtained. In addition, it is possible to offer therapeutic intervention earlier to patients who are hesitant to receive treatment with an anti-PD-1 antibody drug/anti-PD-L1 antibody drug, and to increase a therapeutic effect (for example, inhibition of cancer progression or exacerbation).

### 3. Kit for predicting effect of anti-PD-1 antibody/anti-PD-L1 antibody therapy

The present invention also provides a kit for predicting the effect of anti-PD-1 antibody/anti-PD-L1 antibody therapy. The kit can be used to conveniently carry out the effect prediction method of the present invention. The effect prediction kit of the present invention includes an ISLR detection reagent as an essential element. An appropriate reagent is selected according to the detection means. For example, when the detection means is *in situ* hybridization, the effect prediction kit includes an *in situ* hybridization probe targeting ISLR mRNA as a main component of the effect prediction kit. The *in situ* hybridization probe can be prepared by a conventional method.

On the other hand, when the effect prediction kit employs immunohistochemistry as the detection means, a substance showing specific bindingness to ISLR protein (hereinafter referred to as "binding molecule") is included as the ISLR detection reagent in the effect prediction kit. Examples of the binding molecule include antibodies, nucleic acid aptamers and peptide aptamers that specifically recognize ISLR protein. The type and origin of the binding molecule are not particularly limited as long as it has specific bindingness to ISLR protein. Further, in the case of an antibody, any of a polyclonal antibody, an oligoclonal antibody (a mixture of several to several tens of antibodies), and a monoclonal antibody may be used. As the polyclonal antibody or oligoclonal antibody, an anti-serum-derived IgG fraction obtained by animal immunization and, additionally, an antibody affinity-purified using an antigen can be used. The antibody may be an antibody fragment such as Fab, Fab', F(ab')₂, scFv, or dsFv antibody.

The binding molecule can be prepared by a conventional method. If a commercially available product is available, the commercially available product may be used. For example, an antibody can be prepared using an immunological technique, a phage display method, a ribosome display method, or the like. Preparation of a polyclonal antibody by the immunological technique can be performed by the following procedures. An antigen (ISLR or a part thereof) is prepared and used to immunize an animal such as a mouse, rat, or rabbit. An antigen can be obtained by purifying a biological sample. Alternatively, a recombinant antigen can be used. The recombinant antigen is prepared, for example, by introducing a gene encoding ISLR (which may be a part of the gene) into an appropriate host using a vector and expressing it in the obtained recombinant cell.

In order to enhance the immunity-inducing action, an antigen to which a carrier protein is bound may be used. As the carrier protein, KLH (Keyhole Limpet Hemocyanin), BSA (Bovine Serum Albumin), OVA (Ovalbumin) or the like is used. A carbodiimide method, a glutaraldehyde method, a diazo condensation method, an MBS (maleimidobenzoyloxysuccinimide) method, or the like can be used for binding of the carrier protein. On the other hand, an antigen in which ISLR or a part thereof is expressed as a fusion protein with GST, β-galactosidase, maltose binding protein, or histidine (His) tag can also be used. Such a fusion protein can be conveniently purified by a general-purpose method.

According to need, immunization is repeated, and blood is collected when the antibody titer rises sufficiently, and subjected to centrifugation or the like so that serum is obtained. The obtained antiserum is affinity-purified to prepare a polyclonal antibody.

On the other hand, a monoclonal antibody can be prepared by the following procedures. First, the immunization operation is performed in the same procedures as described above. Immunization is repeated according to need, and antibody-producing cells are extracted from the immunized animal when the antibody titer rises sufficiently. Next, the obtained antibody-producing cells are fused with myeloma cells to prepare hybridomas. Then, the hybridomas are made into monoclonal, and then a clone for producing an antibody having high specificity for the target protein is selected. A culture solution of the selected clone is purified, so that the target antibody is obtained. On the other hand, the target antibody can also be obtained by proliferating the hybridomas to a desired number or more, transplanting them into the abdominal cavity of an animal (e.g., mouse), proliferating them in the ascites, and purifying the ascites. Affinity chromatography using protein G, protein A, or the like is preferably used for the purification of the culture solution or the ascites. Affinity chromatography in which the antigen is immobilized can also be used. Furthermore, methods such as ion exchange chromatography, gel filtration chromatography, ammonium sulfate fractionation, and centrifugation can also be used. These methods may be used alone or combined arbitrarily.

Various modifications can be applied to the obtained antibody, on the condition that the antibody retains specific bindingness to ISLR. Such a modified antibody may be used as the ISLR detection reagent.

When a labeled antibody is used as the specific binding molecule, it is possible to directly detect the amount of the bound antibody using the amount of the label as an index. Therefore, a more convenient examination method can be constructed. However, on the other hand, there is a problem that the detection sensitivity is generally low, in addition to the need to prepare an antibody to which a labeling substance is bound. Therefore, it is preferable to use an indirect detection method such as a method using a secondary antibody to which a labeling substance is bound or a method using a polymer in which a secondary antibody and a labeling substance are bound. The secondary antibody here is an antibody having specific bindingness to an antibody specific to ISLR protein. For example, when the antibody specific to ISLR protein is prepared as a rabbit antibody, an anti-rabbit IgG antibody can be used as the secondary antibody. Labeled secondary antibodies that can be used against various types of antibodies such as rabbit, goat, and mouse antibodies are commercially available (for example, available from Funakoshi Co., Ltd. and Cosmo Bio Co., Ltd.). A suitable labeled secondary antibody can be appropriately selected depending on the reagent of the present invention, and used.

Examples of the labeling substance include enzymes such as peroxidase, microperoxidase, horseradish peroxidase (HRP), alkaline phosphatase, β-D-galactosidase, glucose oxidase, and glucose-6-phosphate dehydrogenase; fluorescent substances such as fluorescein isothiocyanate (FITC), tetramethylrhodamine isothiocyanate (TRITC), and europium; chemiluminescent substances such as luminol, isoluminol, and acridinium derivatives; coenzymes such as NAD; biotin; and radioactive substances such as ¹³¹I and ¹²⁵I.

Other reagents (buffer solution, reaction reagent, labeling reagent, coloring reagent, etc.), containers, devices, and the like used when carrying out the effect prediction method of the present invention may be included in the kit of the present invention. In addition, it is preferable to incorporate the biomarker molecule of the present invention or a fragment thereof as a standard sample in the kit. In addition, an instruction manual is usually attached to the effect prediction kit of the present invention.

### [Examples]

The following studies were conducted with the aim of developing a marker for predicting the effect of anti-PD-1 antibody/anti-PD-L1 antibody therapy.

### 1. Expression of ISLR in lung cancer tissue specimen (surgical specimen)

### (1) Method

Specimens obtained from patients who received resection surgery for lung cancer were used to examine the expression of ISLR by RNA *in situ* hybridization using RNAscope (registered trademark) available from ACD (Advanced Cell Diagnostics). Four appropriate sites were selected from a peripheral part except a central part and a necrotic part for squamous cell carcinoma and from an infiltration part for adenocarcinoma and other types of cancers, so that the observation areas did not overlap, and observed with a 40x objective lens. The procedures 1 to 13 of RNA *in situ* hybridization using RNAscope (registered trademark) (the same procedures were used in the subsequent examples) will be shown below.
1. Slice a specimen (paraffin block) at 4 µm.
2. Within 7 days after the next day, heat-treat the specimen in an oven at 60°C for 1 hour and deparaffinize the treated specimen with xylene.
3. Dehydrate the deparaffinized specimen with 100% ethanol and air-dry the dehydrated specimen for 5 minutes or more.
4. After treatment with a hydrogen peroxide solution (manufactured by ACD) for 10 minutes, activate the treated specimen with an activation liquid (manufactured by ACD) for 15 to 30 minutes (99°C or higher).
5. Dehydrate the activated specimen with 100% ethanol and air-dry the dehydrated specimen for 5 minutes or more until it is completely dried.
6. Digest the protein with protease (manufactured by ACD) for 30 minutes at 40°C.
7. Probe hybridization at 40°C for 2 to 3 hours using a target probe for ISLR (manufactured by ACD).
8. Store the specimen in 5 x SSC until the next day.
9. Under the condition of 40°C, sequentially perform treatment with Amp1 (manufactured by ACD) for 30 minutes, treatment with Amp2 (manufactured by ACD) for 15 minutes, treatment with Amp3 (manufactured by ACD) for 30 minutes, and treatment with Amp4 (manufactured by ACD) for 15 minutes.
10. At room temperature, perform treatment with Amp5 (ACD) for 30 to 60 minutes and treatment with Amp6 (ACD) for 15 minutes.
11. Perform treatment with DAB for 10 to 15 minutes to develop a color.
12. After counterstaining with H&E, dehydrate/clear/embed the specimen with ethanol/xylene.
13. Make observation, cells with 4 dots/cell or more or cluster formation being defined as positive cells.

In each of the procedures 7 to 10, the slide is washed with a washing buffer solution (manufactured by ACD) for 2 minutes × 2 times.

### (2) Results and discussion

The results are shown in Figs. 1 and 2. There were patients with a high expression level (Fig. 1) and patients with a low expression level (Fig. 2). That is, the expression of ISLR was confirmed in cancer-associated fibroblasts of lung cancer.

### 2. Expression of ISLR in cancer-associated fibroblast of lung cancer (biopsy specimen)

### (1) Method

Specimens obtained from patients who were subjected to biopsy for diagnosis of lung cancer were used to examine the expression of ISLR by RNA *in situ* hybridization using RNAscope (registered trademark) available from ACD (Advanced Cell Diagnostics). The method in item 1 above was sufficient, but, for more precise study, up to four sites where tumor cells and cancer-associated fibroblasts coexisted or existed in the vicinity were selected, and observed with a 40 x objective lens. When nucleated cells other than tumor cells were present, it was decided that cancer-associated fibroblasts were also present.

### (2) Results and discussion

The results are shown in Figs. 3 and 4. It was possible to determine the expression of ISLR in the specimens where tumor cells and cancer-associated fibroblasts coexisted or existed in the vicinity, and the presence of patients with a high expression level (Fig. 3) and patients with a low expression level (Fig. 4) was confirmed.

### 3. Correlation between expression of ISLR in cancer-associated fibroblast of lung cancer and effect of anti-PD-1 antibody/anti-PD-L1 antibody therapy

### (1) Method

The methods described in items 1 and 2 above were sufficient, but, for more precise study, the proportion of ISLR-positive cells was calculated in 5% increments in cells remaining after excluding tumor cells, lymphocytes, granulocytes, vascular endothelial cells, and macrophages from all the nucleated cells as much as possible (referred to as "all fibroblasts") found in tumor tissues in visual fields (92792.53 µm² per visual field) observed in the experiments in items 1 and 2 above. The expression was defined as high when the "proportion of ISLR-positive cells in all fibroblasts" was 20% or more, and defined as low when the proportion was less than 20%. However, cells whose type was difficult to identify were all counted as population parameters, and positive signals in which no nucleus could be identified were not counted.

The expressions of ISLR were classified into two groups with high and low expression levels as described above, and determined in terms of tumor reduction effect* (determination of iCR, iPR, iSD, or iUPD), according to iRECIST (Seymour L, Bogaerts J, Perrone A, Ford R, Schwartz LH, Mandrekar S, Lin NU, Litiere S, Dancey J, Chen A et al. (2017) iRECIST: guidelines for response criteria for use in trials testing immunotherapeutics. Lancet Oncology, 18(3):e143-e152), from image records linked to medical records. The correlation between the level (high or low) of ISLR expression and the proportion of patients who achieved iCR or iPR in iBOR* (best overall response) was examined. Moreover, prognosis information such as survival status and disease progression status of the patients was confirmed from the medical records to investigate the correlation between the level (high or low) of ISLR expression and the prognosis. Hereinafter, a method for determining the tumor reduction effect using iRECIST will be described below. The relationship between the ISLR expression levels and the background of the patients is shown in Fig. 5-1 (only for patients who received the anti-PD-1 antibody therapy) and Fig. 5-2 (including patients who received the anti-PD-1 antibody therapy and patients who received the anti-PD-Ll antibody therapy).

### <Determination of tumor reduction effect using iRECIST>

Baseline evaluation: Tumor lesions were identified by the latest chest or thoracoabdominal contrast-enhanced CT (5 mm slice) performed before the start of the treatment, and classified into "measurable lesions" and "unmeasurable lesions". The tumor diameter was measured on the transverse section image of CT, and was not measured in the sagittal section or coronal section of the three-dimensional construction image.

Definition of measurable lesions: Lesions corresponding to 1) or 2) below were defined as measurable lesions:
1) lesions other than lymph node lesions, satisfying either one of the following requirements (non-lymph node lesions):
   having a maximum diameter of 10 mm or more on CT with a slice thickness of 5 mm or less; and
   having a maximum diameter twice or more the slice thickness on CT with a slice thickness of more than 5 mm;
2) lymph node lesions having a minor diameter of 15 mm or more on CT with a slice thickness of 5 mm or less
   (lymph node lesions having a minor diameter of 10 mm or more and less than 15 mm are unmeasurable lesions, and lymph nodes having a minor diameter of less than 10 mm are not lesions).

All lesions other than the above ones were defined as unmeasurable lesions.

Of the measurable lesions observed at the time of registration, up to 5 lesions were selected in descending order of diameter (major diameter for the non-lymph node lesions and minor diameter for the lymph node lesions) (up to 2 lesions per organ) were selected and defined as target lesions. The selection was made in such a manner that organs with measurable lesions were included as evenly as possible and in consideration of the reproducibility during repeated measurement, i.e., ease of measurement (lesions difficult to measure even though having a large diameter were avoided). For the selected target lesions, the sum of the diameters of all the target lesions (hereinafter, diameter sum) was calculated. The tumor reduction effect was determined according to the following "criteria for determination of effect on target lesions". For non-target lesions, the effect was determined according to separately set effect determination criteria (criteria for determination of effect on non-target lesions).

### Criteria for determination of effect on target lesions

iCR (complete response): cases where all the non-lymph node target lesions disappear and the minor diameters of all the lymph node target lesions were less than 10 mm
iPR (partial response): cases where the diameter sum of the target lesions was 30% or more smaller than the baseline diameter sum
iSD (stable disease): cases where tumor shrinkage corresponding to PR or tumor growth corresponding to PD was not observed
iUPD (progressive disease): cases where the diameter sum of the target lesions was 20% or more larger than the previous smallest diameter sum and increased by 5 mm or more in terms of the absolute value, or cases where a new lesion appeared.

### Criteria for determination of effect on non-target lesions

iCR: cases where all the non-lymph node non-target lesions disappear and the minor diameters of all the lymph node non-target lesions were less than 10 mm
Non-iCR/Non-iUPD: cases where one or more non-lymph node non-target lesions did not disappear, or one or more lymph node non-target lesions had a minor diameter of 10 mm or more, and no clear exacerbation was observed
iUPD: "clear exacerbation of non-target lesions (: increase in tumor volume of the non-target lesions far exceeding reduction in tumor volume of the target lesions)"
NE (unevaluable): cases where no examination could be performed for some reason, or cases where the effect could not be determined to be any of CR, Non-CR/non-PD, and PD (there was no corresponding case in this study)

The overall response was determined in consideration of the combination of the effect on the target lesions, the effect on the non-target lesions, and the presence or absence of new lesion appearance, and the overall response obtained 4 weeks or more before that. When the immediately preceding overall response was determined as iUPD, and further tumor growth or further appearance of a new lesion was confirmed, the effect was determined as iCPD (confirmed progressive disease). Concerning the best overall response (iBOR), the comprehensive evaluation was evaluated over time, and the best effect was determined as the best overall response. At that time, iUPD confirmed before achievement of iCR, iPR or iSD was ignored.

### (2) Results and discussion

The results are shown in Fig. 6-1 (evaluation in patients who received the anti-PD-1 antibody therapy) and Fig. 6-2 (evaluation in patients including patients who received the anti-PD-1 antibody therapy and patients who received the anti-PD-Ll antibody therapy). It was confirmed that a better effect can be obtained in patients with high expression of ISLR. Specifically, it was shown that high expression of ISLR in cancer-associated fibroblasts correlates with the objective response percentage, and that this is a factor that defines the responsiveness to treatment, that is, an effect prediction marker.

### 4. Correlation between expression of ISLR in cancer-associated fibroblast of lung cancer and survival of patient who received anti-PD-1 antibody/anti-PD-L1 antibody therapy

### (1) Method

Similarly to item 3 above, the proportion of ISLR-positive cells was calculated in 5% increments in cells remaining after excluding tumor cells, lymphocytes, granulocytes, vascular endothelial cells, and macrophages from all the nucleated cells as much as possible (all fibroblasts) found in tumor tissues in visual fields (92792.53 µm² per visual field) observed in the experiments in items 1 and 2 above. The expression was defined as high when the "proportion of ISLR-positive cells in all fibroblasts" was 20% or more, and defined as low when the proportion was less than 20%. However, cells whose type was difficult to identify were all counted, and positive signals in which no nucleus could be identified were not counted.

The expressions of ISLR were classified into two groups with high and low expression levels as described above, and prognosis information such as survival status and disease progression status of the patients was confirmed from the medical records to investigate the correlation between the level (high or low) of ISLR expression and the overall survival period or the progression-free survival period.

### (2) Results and discussion

The results are shown in Fig. 7-1 (evaluation in patients who received the anti-PD-1 antibody therapy) and Fig. 7-2 (evaluation in patients including patients who received the anti-PD-1 antibody therapy and patients who received the anti-PD-Ll antibody therapy), and Fig. 8-1 (evaluation in patients who received the anti-PD-1 antibody therapy) and Fig. 8-2 (evaluation in patients including patients who received the anti-PD-1 antibody therapy and patients who received the anti-PD-Ll antibody therapy). It was revealed that a better prognosis can be obtained in patients with high expression of ISLR. That is, high expression of ISLR in cancer-associated fibroblasts correlated with the prognosis. This result supports the result that high expression of ISLR is an effect prediction marker, which was demonstrated in the experiment in item 3 above.

### 5. Relationship between PD-L1 expression and effect of anti-PD-1 antibody/anti-PD-L1 antibody therapy (comparative example)

The surgical and biopsy specimens used in this study were used to perform immunostaining (using an anti-PD-Ll antibody (Clone:22C3)) using the same automatic staining device and method as those for the companion diagnostic drug for KEYTRUDA, thereby investigating the relationship between the expression level of PD-L1 in cancer cells and the effect of anti-PD-1 antibody/anti-PD-L1 antibody therapy. As a result, no correlation was observed between the expression level of PD-L1 and the effect (Fig. 9) or the overall survival period (Fig. 10) or the progression-free survival period (Fig. 11).

### 6. Correlation between expression of ISLR in cancer-associated fibroblast of lung cancer patient who did not receive anti-PD-1 antibody/anti-PD-L1 antibody therapy and survival after surgery

### (1) Method

The expressions of ISLR were classified into two groups with high and low expression levels, in the same manner as described in item 4 above, in a group of patients (Fig. 12) different from those in item 1 above, and prognosis information such as survival status and disease progression status of the patients was confirmed from the medical records to investigate the correlation between the level (high or low) of ISLR expression and the overall survival period or the disease-free survival period.

### (2) Results and discussion

The results are shown in Figs. 13 to 16. It was found that the prognosis further worsens when no anti-PD-1 antibody/anti-PD-L1 antibody therapy was performed on patients with high expression of ISLR. That is, high expression of ISLR in cancer-associated fibroblasts does not correlate with a good prognosis. This result strongly supports that ISLR expression is not a prognosis prediction marker, but is extremely useful as a marker for predicting the therapeutic effect of the anti-PD-1 antibody/anti-PD-L1 antibody therapy.

### 7. Correlation between ISLR expression and prognosis (comparative example)

The correlation between the expression of ISLR and the prognosis was investigated using the application OncoLnc (http://www.oncolnc.org/ Anaya J. OncoLnc: linking TCGA survival data to mRNAs, miRNAs, and IncRNAs. Peer J Computer Science. 2016;2:e67) for confirming the correlation with the prognosis based on the expression level (high or low) of the target gene from the database of The Cancer Genome Atlas (TCGA) possessed by the U.S. NIH. As a result, there was no significant association between the expression of ISLR and the overall survival period in either of the cases of adenocarcinoma (Fig. 17) and squamous cell carcinoma (Fig. 18).

### 8. Conclusion

It was shown that the expression level of ISLR can be used to predict the effect of anti-PD-1 antibody/anti-PD-L1 antibody therapy with high sensitivity, even in a population for which the effect could not be predicted with the marker PD-L1 which is said to be associated with the responsiveness to the anti-PD-1 antibody/anti-PD-L1 antibody (Figs. 9 to 11). Also, from the prognostic information on the patient population which received no anti-PD-1 antibody/anti-PD-L1 antibody therapy (Figs. 13 to 16) and the lung cancer patient population from the TCGA database possessed by the U.S. NIH (Figs. 17 and 18), it was shown that the level (high or low) of ISLR expression is not a good prognosis prediction marker. The use of the expression of ISLR as an index enables selection of patients for whom the effect can hardly be expected, which is important and useful particularly in the field of cancer treatment.

### [Industrial Applicability]

The present invention provides a means for predicting the effect of anti-PD-1 antibody/anti-PD-L1 antibody therapy. According to the present invention, the effect of anti-PD-1 antibody/anti-PD-L1 antibody therapy can be predicted with high sensitivity. A more appropriate treatment policy can be formed for each patient using the prediction results. In particular, the advantage that patients who do not respond to the anti-PD-1 antibody/anti-PD-L1 antibody therapy can be identified and excluded from the treatment targets reduces the burden on the patients and increases the therapeutic effect (due to early selection of another treatment), and, additionally, greatly contributes to the medical economy.

The present invention is not limited to the above description of the embodiments and examples of the present invention at all. Various modifications that can be easily achieved by those skilled in the art without departing from the claims also fall within the scope of the present invention. The contents of the articles, patent laid-open publications, patent publications, and the like specified herein shall be cited by incorporation in their entity.

## Claims

1. A biomarker for predicting an effect of anti-PD-1 antibody/anti-PD-L1 antibody therapy, comprising an immunoglobulin superfamily containing leucine-rich repeat (ISLR).

2. A method for predicting an effect of anti-PD-1 antibody/anti-PD-L1 antibody therapy, comprising using expression of ISLR in cells in a tumor tissue derived from a subject as an index.

3. The method according to claim 2, wherein the cells are nucleated cells other than tumor cells.

4. The method according to claim 2, wherein the cells are fibroblasts.

5. The effect prediction method according to any one of claims 2 to 4, comprising the following steps (1) to (3):
(1) a step of preparing a tumor tissue specimen collected from a subject;
(2) a step of examining expression of ISLR in cells in the tumor tissue specimen; and
(3) a step of predicting the effect of anti-PD-1 antibody/anti-PD-L1 antibody therapy based on the result of step (2), wherein high expression of ISLR indicates that the anti-PD-1 antibody/anti-PD-L1 antibody therapy can be expected to be effective.

6. The effect prediction method according to claim 5, wherein, in step (2), an expression level of ISLR is determined based on the number of ISLR-positive cells and/or a ratio of ISLR-positive cells to ISLR-negative cells.

7. The effect prediction method according to claim 5 or 6, further comprising the following step (4):
(4) a step of excluding a subject for whom the anti-PD-1 antibody/anti-PD-L1 antibody therapy cannot be expected to be effective from treatment targets based on a prediction result.

8. The effect prediction method according to claim 5 or 6, further comprising the following step (4'):
(4') a step of determining or changing a treatment policy for a subject based on a prediction result.

9. The effect prediction method according to any one of claims 2 to 8, wherein the subject is a patient suffering from malignant melanoma, non-small cell lung cancer, renal cell carcinoma, head and neck cancer, gastric cancer, Hodgkin lymphoma, urothelial cancer, breast cancer, pancreatic cancer, or colon cancer.

10. A kit for predicting an effect of anti-PD-1 antibody/anti-PD-L1 antibody therapy, comprising an ISLR detection reagent.

11. The effect prediction kit according to claim 10, wherein the ISLR detection reagent is an *in situ* hybridization probe.

12. The effect prediction kit according to claim 10, wherein the ISLR detection reagent is an anti-ISLR antibody.


### [Sequence Listing]

SEQUENCE LISTING

<110>  NATIONAL UNIVERSITY CORPORATION NAGOYA UNIVERSITY

<120>  BIOMARKER FOR PREDICTING EFFECT OF ANTI-PD-1 ANTIBODY /
       ANTI-PD-L1 ANTIBODY THERAPY

<130>  NU17013P

<150>  JP P2018-024556
<151>  2018-02-14

<160>  2

<170>  PatentIn version 3.5

<210>  1
<211>  428
<212>  PRT
<213>  Homo sapiens

<400>  1

Met Gln Glu Leu His Leu Leu Trp Trp Ala Leu Leu Leu Gly Leu Ala
1               5                   10                  15

Gln Ala Cys Pro Glu Pro Cys Asp Cys Gly Glu Lys Tyr Gly Phe Gln
            20                  25                  30

Ile Ala Asp Cys Ala Tyr Arg Asp Leu Glu Ser Val Pro Pro Gly Phe
        35                  40                  45

Pro Ala Asn Val Thr Thr Leu Ser Leu Ser Ala Asn Arg Leu Pro Gly
    50                  55                  60

Leu Pro Glu Gly Ala Phe Arg Glu Val Pro Leu Leu Gln Ser Leu Trp
65                  70                  75                  80

Leu Ala His Asn Glu Ile Arg Thr Val Ala Ala Gly Ala Leu Ala Ser
                85                  90                  95

Leu Ser His Leu Lys Ser Leu Asp Leu Ser His Asn Leu Ile Ser Asp
            100                 105                 110

Phe Ala Trp Ser Asp Leu His Asn Leu Ser Ala Leu Gln Leu Leu Lys
        115                 120                 125

Met Asp Ser Asn Glu Leu Thr Phe Ile Pro Arg Asp Ala Phe Arg Ser
    130                 135                 140

Leu Arg Ala Leu Arg Ser Leu Gln Leu Asn His Asn Arg Leu His Thr
145                 150                 155                 160

Leu Ala Glu Gly Thr Phe Thr Pro Leu Thr Ala Leu Ser His Leu Gln
                165                 170                 175

Ile Asn Glu Asn Pro Phe Asp Cys Thr Cys Gly Ile Val Trp Leu Lys
            180                 185                 190

Thr Trp Ala Leu Thr Thr Ala Val Ser Ile Pro Glu Gln Asp Asn Ile
        195                 200                 205

Ala Cys Thr Ser Pro His Val Leu Lys Gly Thr Pro Leu Ser Arg Leu
    210                 215                 220

Pro Pro Leu Pro Cys Ser Ala Pro Ser Val Gln Leu Ser Tyr Gln Pro
225                 230                 235                 240

Ser Gln Asp Gly Ala Glu Leu Arg Pro Gly Phe Val Leu Ala Leu His
                245                 250                 255

Cys Asp Val Asp Gly Gln Pro Ala Pro Gln Leu His Trp His Ile Gln
            260                 265                 270

Ile Pro Ser Gly Ile Val Glu Ile Thr Ser Pro Asn Val Gly Thr Asp
        275                 280                 285

Gly Arg Ala Leu Pro Gly Thr Pro Val Ala Ser Ser Gln Pro Arg Phe
    290                 295                 300

Gln Ala Phe Ala Asn Gly Ser Leu Leu Ile Pro Asp Phe Gly Lys Leu
305                 310                 315                 320

Glu Glu Gly Thr Tyr Ser Cys Leu Ala Thr Asn Glu Leu Gly Ser Ala
                325                 330                 335

Glu Ser Ser Val Asp Val Ala Leu Ala Thr Pro Gly Glu Gly Gly Glu
            340                 345                 350

Asp Thr Leu Gly Arg Arg Phe His Gly Lys Ala Val Glu Gly Lys Gly
        355                 360                 365

Cys Tyr Thr Val Asp Asn Glu Val Gln Pro Ser Gly Pro Glu Asp Asn
    370                 375                 380

Val Val Ile Ile Tyr Leu Ser Arg Ala Gly Asn Pro Glu Ala Ala Val
385                 390                 395                 400

Ala Glu Gly Val Pro Gly Gln Leu Pro Pro Gly Leu Leu Leu Leu Gly
                405                 410                 415

Gln Ser Leu Leu Leu Phe Phe Phe Leu Thr Ser Phe
            420                 425

<210>  2
<211>  2312
<212>  DNA
<213>  Homo sapiens

<400>  2
aagcagttgt tttgctggaa ggagggagtg cgcgggctgc cccgggctcc tccctgccgc      60

ctcctctcag tggatggttc caggcaccct gtctggggca gggagggcac aggcctgcac     120

atcgaaggtg gggtgggacc aggctgcccc tcgccccagc atccaagtcc tcccttgggc     180

gcccgtggcc ctgcagactc tcagggctaa ggtcctctgt tgctttttgg ttccacctta     240

gaagaggctc cgcttgacta agagtagctt gaaggaggca ccatgcagga gctgcatctg     300

ctctggtggg cgcttctcct gggcctggct caggcctgcc ctgagccctg cgactgtggg     360

gaaaagtatg gcttccagat cgccgactgt gcctaccgcg acctagaatc cgtgccgcct     420

ggcttcccgg ccaatgtgac tacactgagc ctgtcagcca accggctgcc aggcttgccg     480

gagggtgcct tcagggaggt gcccctgctg cagtcgctgt ggctggcaca caatgagatc     540

cgcacggtgg ccgccggagc cctggcctct ctgagccatc tcaagagcct ggacctcagc     600

cacaatctca tctctgactt tgcctggagc gacctgcaca acctcagtgc cctccaattg     660

ctcaagatgg acagcaacga gctgaccttc atcccccgcg acgccttccg cagcctccgt     720

gctctgcgct cgctgcaact caaccacaac cgcttgcaca cattggccga gggcaccttc     780

accccgctca ccgcgctgtc ccacctgcag atcaacgaga accccttcga ctgcacctgc     840

ggcatcgtgt ggctcaagac atgggccctg accacggccg tgtccatccc ggagcaggac     900

aacatcgcct gcacctcacc ccatgtgctc aagggtacgc cgctgagccg cctgccgcca     960

ctgccatgct cggcgccctc agtgcagctc agctaccaac ccagccagga tggtgccgag    1020

ctgcggcctg gttttgtgct ggcactgcac tgtgatgtgg acgggcagcc ggcccctcag    1080

cttcactggc acatccagat acccagtggc attgtggaga tcaccagccc caacgtgggc    1140

actgatgggc gtgccctgcc tggcacccct gtggccagct cccagccgcg cttccaggcc    1200

tttgccaatg gcagcctgct tatccccgac tttggcaagc tggaggaagg cacctacagc    1260

tgcctggcca ccaatgagct gggcagtgct gagagctcag tggacgtggc actggccacg    1320

cccggtgagg gtggtgagga cacactgggg cgcaggttcc atggcaaagc ggttgaggga    1380

aagggctgct atacggttga caacgaggtg cagccatcag ggccggagga caatgtggtc    1440

atcatctacc tcagccgtgc tgggaaccct gaggctgcag tcgcagaagg ggtccctggg    1500

cagctgcccc caggcctgct cctgctgggc caaagcctcc tcctcttctt cttcctcacc    1560

tccttctagc cccacccagg gcttccctaa ctcctcccct tgcccctacc aatgcccctt 1620

taagtgctgc aggggtctgg ggttggcaac tcctgaggcc tgcatgggtg acttcacatt 1680

ttcctacctc tccttctaat ctcttctaga gcacctgcta tccccaactt ctagacctgc 1740

tccaaactag tgactaggat agaatttgat cccctaactc actgtctgcg gtgctcattg 1800

ctgctaacag cattgcctgt gctctcctct caggggcagc atgctaacgg ggcgacgtcc 1860

taatccaact gggagaagcc tcagtggtgg aattccaggc actgtgactg tcaagctggc 1920

aagggccagg attgggggaa tggagctggg gcttagctgg gaggtggtct gaagcagaca 1980

gggaatggga gaggaggatg ggaagtagac agtggctggt atggctctga ggctccctgg 2040

ggcctgctca agctcctcct gctccttgct gttttctgat gatttggggg cttgggagtc 2100

cctttgtcct catctgagac tgaaatgtgg ggatccagga tggccttcct tcctcttacc 2160

cttcctccct cagcctgcaa cctctatcct ggaacctgtc ctccctttct ccccaactat 2220

gcatctgttg tctgctcctc tgcaaaggcc agccagcttg ggagcagcag agaaataaac 2280

agcatttctg atgccaaaaa aaaaaaaaaa aa 2312